# EUROPEAN PATENT APPLICATION

(11) **EP 4 130 236 A1**
(43) Date of publication of application: **08.02.2023**
(21) Application number: 21781087.8
(22) Date of filing: 01.04.2021
(51) Int. Cl.: C12M 3/00, C12N 5/02

(54) **METHOD FOR PRODUCING CELL-CONTAINING EXTRACELLULAR MATRIX, CELL CULTURE METHOD, DEVICE FOR PRODUCING CELL-CONTAINING EXTRACELLULAR MATRIX AND CONTROL PROGRAM**

(30) Priority: 03.04.2020 JP 2020067519
(71) Applicant: JSR Corporation, Tokyo 105-8640 (JP)
(72) Inventor: SUEMASA, Daichi, Tokyo 105-8640 (JP); HAYASHI, Eiji, Tokyo 105-8640 (JP)
(74) Representative: TBK
(86) International application number: PCT/JP2021/014208
(87) International publication number: WO 2021/201243

(57) **Abstract**

A method for producing a cell-containing extracellular matrix is provided, the method including preparing an extracellular matrix solution which contains (i) an extracellular matrix precursor and (ii) cells or a cell mass inside a pipette having a tip opening portion; discharging the extracellular matrix solution to form a drop of the extracellular matrix solution at the tip opening portion of the pipette; bringing the tip opening portion of the pipette close to a culture surface of a cell culture container to place the drop on the culture surface while avoiding bringing the tip opening portion of the pipette into contact with the culture surface; moving the tip opening portion of the pipette away from the culture surface to separate the drop from the tip opening portion of the pipette; and gelating the extracellular matrix solution to form a cell-containing extracellular matrix.

## Description

### Field of the Invention

The present invention relates to a method for producing a cell-containing extracellular matrix, a cell culture method, and a device for producing a cell-containing extracellular matrix and a control program thereof. Priority is claimed on Japanese Patent Application No. 2020-067519, filed April 3, 2020, the content of which is incorporated herein by reference.

### Description of Related Art

In cell culture, it is known that, due to differences in an operator's manipulation, the same result is not obtained in a large number of cases, even in a case where the cell culture is carried out with the same protocol. For example, it is almost impossible to produce a plurality of extracellular matrices containing cells having almost the same size and shape by a manual operation using a pipette.

In addition, in a case where cell culture is carried out using extracellular matrices with variations in size and shape, it is known that proliferation or differentiation differs among cells in each extracellular matrix (see, for example, Non-Patent Documents 1 to 4) since variations occur in oxygen or carbon dioxide supplied to the cells in each extracellular matrix and nutritional components from the culture medium.

In a case where the size of the extracellular matrix is reduced, oxygen, carbon dioxide, and nutritional components from the culture medium can be sufficiently supplied to the inside of the extracellular matrix, and thus the variations can be suppressed. However, it is very difficult to produce a small extracellular matrix by the operator's manipulation.

### Citation List

### [Non-Patent Documents]

[Non-Patent Document 1]
   Wang Y., et al., Three-dimensional biomimetic scaffolds for hepatic differentiation of size-controlled embryoid bodies, J Mater Res., 34 (8), 1371-1380, 2019.
[Non-Patent Document 2]
   Vernon R. B., et al., Adhesion, shape, proliferation, and gene expression of mouse Leydig cells are influenced by extracellular matrix in vitro, Biol Reprod., 44 (1), 157-170, 1991.
[Non-Patent Document 3]
   Huet C., et al., Extracellular matrix regulates ovine granulosa cell survival, proliferation and steroidogenesis: relationships between cell shape and function, J Endocrinol., 169 (2), 347-360, 2001.
[Non-Patent Document 4]
   Kumar G., Freeform fabricated scaffolds with roughened struts that enhance both stem cell proliferation and differentiation by controlling cell shape, Biomaterials., 33 (16), 4022-4030, 2012.

### Summary of the Invention

### Technical Problem

In order to industrialize cell culture, it is necessary to eliminate such differences in an operator's manipulation. For that purpose, the mechanical automation of cell culture is required. An object of the present invention is to provide a method for producing a cell-containing extracellular matrix which is suitable for mechanical automation.

### Solution to Problem

The present invention includes the following embodiments.
[1] A method for producing a cell-containing extracellular matrix, the method including:
   preparing an extracellular matrix solution which contains an extracellular matrix precursor and cells or a cell mass inside a pipette having a tip opening portion;
   discharging the extracellular matrix solution to form a drop of the extracellular matrix solution at the tip opening portion of the pipette;
   bringing the tip opening portion of the pipette close to a culture surface of a cell culture container to place the drop on the culture surface while avoiding bringing the tip opening portion of the pipette into contact with the culture surface;
   moving the tip opening portion of the pipette away from the culture surface to separate the drop from the tip opening portion of the pipette; and
   gelating the extracellular matrix solution to form a cell-containing extracellular matrix.
[2] The method for producing a cell-containing extracellular matrix according to [1], in which a contact angle of water on the culture surface is 40° to 120° at 25°C.
[3] The method for producing a cell-containing extracellular matrix according to [1] or [2], in which at a time of forming the cell-containing extracellular matrix, a temperature of the culture surface is 20°C to 60°C.
[4] The method for producing a cell-containing extracellular matrix according to any one of [1] 1 to [3], in which a temperature of the extracellular matrix solution is 0°C to 10°C.
[5] The method for producing a cell-containing extracellular matrix according to any one of [1] to [4], in which an opening area of the tip opening portion of the pipette is 0.05 mm² to 0.4 mm².
[6] The method for producing a cell-containing extracellular matrix according to any one of [1] to [5], in which in the drop, a ratio of a maximum value of a length in a horizontal direction to a maximum value of a length in a vertical direction (the maximum value of the length in the horizontal direction/the maximum value of the length in the vertical direction) is 0.5 to 5.
[7] The method for producing a cell-containing extracellular matrix according to any one of [1] to [6], in which a maximum value of a length of the drop in a vertical direction is 100 µm to 5,000 µm.
[8] The method for producing a cell-containing extracellular matrix according to any one of [1] to [7], in which the cell culture container is a well plate.
[9] The method for producing a cell-containing extracellular matrix according to any one of [1] to [8],
   in which at a time of preparing the extracellular matrix solution, sucking the extracellular matrix solution into the inside of the pipette while maintaining a state where the tip opening portion of the pipette is below a solution surface of the extracellular matrix solution.
[10] A cell culture method including bringing a cell-containing extracellular matrix formed according to the method for producing a cell-containing extracellular matrix according to any one of [1] to [9] into contact with a culture medium and culturing the cells.
[11] A device for producing a cell-containing extracellular matrix, the device containing:
   an accommodation tank that accommodates an extracellular matrix solution containing an extracellular matrix precursor and cells or a cell mass;
   a pipette control unit including a nozzle to which a pipette having a tip opening portion is mounted, a pump that applies positive pressure or negative pressure to the nozzle, a transfer unit that transfers the nozzle, and a control unit that controls operations of the nozzle, the pump, and the transfer unit; and
   a stage that holds a cell culture container.
[12] A control program for a cell-containing extracellular matrix-producing device, causing a cell-containing extracellular matrix-producing device to form a cell-containing extracellular matrix on a culture surface of a cell culture container, the cell-containing extracellular matrix-producing device including an accommodation tank that accommodates an extracellular matrix solution containing cells or a cell mass in an extracellular matrix precursor, a pipette control unit including a nozzle to which a pipette having a tip opening portion is mounted, a pump that applies positive pressure or negative pressure to the nozzle, a transfer unit that transfers the nozzle, and a control unit that controls operations of the nozzle, the pump, and the transfer unit, and a stage that holds a cell culture container,
   in which in the device for producing a cell-containing extracellular matrix in which an extracellular matrix solution in which cells or a cell mass is suspended is accommodated in the accommodation tank, a pipette having a tip opening portion is mounted on the nozzle, and the cell culture container is placed on the stage,
   in which the control unit is caused to control the transfer unit to move a tip of the pipette mounted on the nozzle below a solution surface of the extracellular matrix solution accommodated in the accommodation tank,
   the control unit is caused to control the pump to suck the extracellular matrix solution into the inside of the pipette,
   the control unit is caused to control the transfer unit to move the pipette that has sucked the extracellular matrix solution to an upper part of the cell culture container,
   the control unit is caused to control the pump to discharge the extracellular matrix solution in the inside of the pipette to form a drop of the extracellular matrix solution at the tip opening portion of the pipette,
   the control unit is caused to control the transfer unit to bring the tip opening portion of the pipette close to the culture surface of the cell culture container and to place the drop on the culture surface while avoiding bringing the tip opening portion of the pipette into contact with the culture surface,
   the control unit is caused to control the transfer unit to move the tip opening portion of the pipette away from the culture surface, to separate the drop from the tip opening portion of the pipette, and
   the extracellular matrix solution is subsequently gelated to form a cell-containing extracellular matrix.

### Advantageous Effects of Invention

According to the present embodiment, it is possible to provide a method for producing a cell-containing extracellular matrix which is suitable for mechanical automation.

### Brief Description of the Drawings

FIG. 1 is a plan view showing an example of a device for producing a cell-containing extracellular matrix.
FIG. 2A is a side view showing an example of a pipette rack.
FIG. 2B is a side view showing an example of a pipette rack.
FIG. 3 is a block diagram showing a configuration of a control unit.
FIG. 4A is a photographic image showing the result of placing drops of a cell-containing extracellular matrix solution in a well of a 6-well plate by an operator's manipulation.
FIG. 4B is a photographic image showing the result of placing drops of the cell-containing extracellular matrix solution in a well of a 6-well plate using the device for producing a cell-containing extracellular matrix.
FIG. 5A is a photographic image showing a state where a drop of an extracellular matrix solution is formed at a tip opening portion of a pipette.
FIG. 5B is a schematic view showing a state where a drop of an extracellular matrix solution is formed at the tip opening portion of a pipette.
FIG. 6A is a photographic image showing the result of placing drops of a cell-containing extracellular matrix solution in a well of a 6-well plate by an operator's manipulation.
FIG. 6B is a photographic image showing the result of placing drops of the cell-containing extracellular matrix solution in a well of a 6-well plate using the device for producing a cell-containing extracellular matrix.
FIG. 6C is a photographic image showing the result of discharging the cell-containing extracellular matrix solution in the vicinity of a well of a 6-well plate to place a drop on the well without forming a drop of the cell-containing extracellular matrix solution at the tip opening portion of a pipette, although the device for producing a cell-containing extracellular matrix was used.

### Detailed Description of the Invention

Hereinafter, embodiments of the present invention will be described in detail; however, the present invention is not limited to the following embodiments. In addition, in the drawings, the same or corresponding parts are designated by the same or corresponding reference numerals, and the description thereof will not be duplicated. The dimensional ratio in each figure may be exaggerated for description and thus may not necessarily match the actual dimensional ratio. The numerical values in the specification indicate numerical values in the standard state. In the present specification, the standard state means a state where the temperature is about 25°C and the atmospheric pressure is about 101 kPa in terms of absolute pressure.

### [Method for Producing Cell-containing Extracellular Matrix]

A method for producing a cell-containing extracellular matrix according to an embodiment includes preparing an extracellular matrix solution which contains an extracellular matrix precursor and cells or a cell mass inside a pipette having a tip opening portion; discharging the extracellular matrix solution to form a drop of the extracellular matrix solution at the tip opening portion of the pipette; bringing the tip opening portion of the pipette close to a culture surface of a cell culture container to place the drop on the culture surface while avoiding bringing the tip opening portion of the pipette into contact with the culture surface; moving the tip opening portion of the pipette away from the culture surface to separate the drop from the tip opening portion of the pipette; and gelating the extracellular matrix solution to form a cell-containing extracellular matrix.

The production method according to the present embodiment is suitable for automation, and as a result, a plurality of cell-containing extracellular matrices having a uniform size and shape can be produced. The production method according to the present embodiment is preferably carried out using a device for producing a cell-containing extracellular matrix described later. In a case of using the device for producing a cell-containing extracellular matrix according to the present embodiment, it is easy to produce a plurality of cell-containing extracellular matrices having a uniform size and shape, rapidly with high accuracy, as compared with a case of producing a cell-containing extracellular matrix by the operator's manual operation.

The extracellular matrix is a gel that serves as a scaffold for cells in cell culture. In the present specification, the extracellular matrix solution refers to a solution containing cells or a cell mass in an extracellular matrix precursor. The extracellular matrix solution is preferably a solution in which cells or a cell mass is suspended in an extracellular matrix precursor. The extracellular matrix solution is a sol. Examples of a commercially available extracellular matrix precursor include Matrigel (product name of Corning Incorporated), human laminin (product name of Sigma-Aldrich Co., LLC), and the product name "Mebiol Gel" (manufactured by Mebiol Inc.).

The extracellular matrix precursor is a sol containing a component (hereinafter, an "extracellular matrix component") that is crosslinked by a stimulus such as light or heat and a liquid medium. Examples of the liquid medium include a physiological saline solution and a buffer solution. The extracellular matrix precursor is gelated to form an extracellular matrix.

Examples of the extracellular matrix component include a component contained in the basement membrane and a glycoprotein present in the intercellular space. Examples of the component contained in the basement membrane include type IV collagen, laminin, heparan sulfate proteoglycan, and entactin. Examples of the glycoprotein present in the intercellular space include collagen, laminin, entactin, fibronectin, fibrinogen, and heparin sulfate.

Examples of the extracellular matrix component also include an artificial water-soluble polymer. Examples of the artificial water-soluble polymer include carboxymethyl cellulose, a polymer containing an azobenzene group and a cyclodextrin group, and calcium alginate.

The cell mass refers to a mass in which cells in a range of 2 to 100 cells are adhered. The cells contained in the cell mass may be derived from a single cell type or a plurality of cell type.

Hereinafter, each step of the production method according to the present embodiment will be described. In the inside of a pipette having a tip opening portion, an extracellular matrix solution containing cells or a cell aggregate mass (hereinafter, also referred to as "cells or the like") is prepared and preferably sucked in an extracellular matrix precursor. In addition, the extracellular matrix solution is maintained under conditions in which it is not gelated. Therefore, the temperature of the extracellular matrix solution sucked into the inside of the pipette is generally 0°C to 10°C. In the present specification, the pipette refers to a tube in which the inner diameter of one tip side is smaller than the inner diameter of the other thereof.

At the time of sucking the extracellular matrix solution, it is preferable to suck the extracellular matrix solution into the inside of the pipette while maintaining a state where the tip opening portion of the pipette is below the solution surface of the extracellular matrix solution. In a case of sucking in this way, it is possible to prevent air bubbles from mixedly entering the inside of the pipette.

In a case where air bubbles mixedly enter the inside of the pipette, a cell-containing extracellular matrix with defects is formed due to the air bubbles. In a case of sucking the extracellular matrix solution into the inside of the pipette while maintaining a state where the tip opening portion of the pipette is below a solution surface of the extracellular matrix solution, it is possible to prevent air bubbles from mixedly entering the inside of the pipette. As a result, a cell-containing extracellular matrix without defects can be produced.

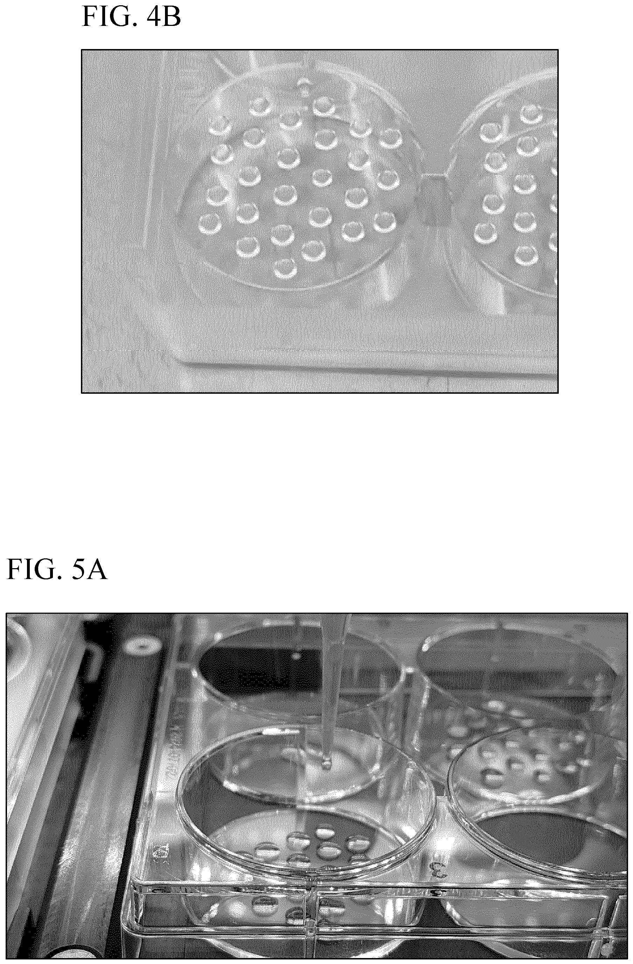

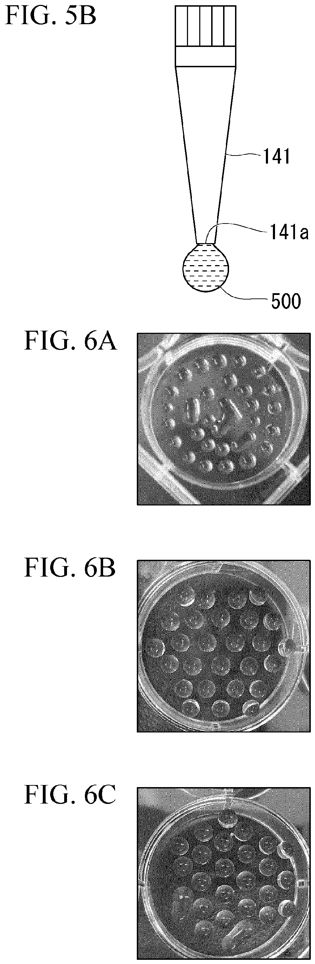

Subsequently, the extracellular matrix solution is discharged from the pipette, whereby a drop of the extracellular matrix solution is formed at the tip opening portion of the pipette. The drop is formed by the surface tension of the extracellular matrix solution. FIG. 5A is a photographic image showing a state where a drop 500 of an extracellular matrix solution is formed at a tip opening portion 141a of a pipette 141. FIG. 5B is a schematic view showing a state where the drop 500 of an extracellular matrix solution is formed at the tip opening portion 141a of the pipette 141. As shown in FIGS. 5A and 5B, the drop 500 is held at the tip opening portion 141a.

In the general manipulation, in order to form an extracellular matrix at a predetermined position on the culture surface of the cell culture container, an extracellular matrix solution sucked into the inside of the pipette was discharged, and at the same time, the discharged extracellular matrix solution was placed on the culture surface of the cell culture container to form an extracellular matrix. However, in this method, it was difficult to form the shape of the extracellular matrix into a dome shape. The inventors of the present invention speculate that the cause of this is presumed to be because the surface tension of the extracellular matrix solution cannot withstand the momentum of discharge and the extracellular matrix solution spreads on the culture surface.

On the other hand, the inventors of the present invention found that in a case of forming once a drop of the extracellular matrix solution at the tip opening portion of the pipette and then placing the formed drop on the culture surface of the cell culture container, it is possible to produce a cell-containing extracellular matrix having a dome shape.

In the drop, the length in the vertical direction is preferably 10 µm to 5,000 µm. In addition, in the drop, it is preferable that the ratio of the maximum value of the length in the horizontal direction to the maximum value of the length in the vertical direction (the maximum value of the length in the horizontal direction/the maximum value of the length in the vertical direction) be 0.5 to 5 and preferably 0.6 to 2. In a case where the drop has the above size and shape, a cell-containing extracellular matrix having a preferred size and shape is obtained. The opening area of the tip opening portion of the pipette is preferably 0.05 mm² to 0.4 mm². In a case where the opening area of the tip opening portion of the pipette is within the above range, a drop having the above size and shape can be formed.

Subsequently, the tip opening portion of the pipette is brought close to the culture surface of the cell culture container, and the drop is placed on the culture surface while avoiding bringing the tip opening portion of the pipette into contact with the culture surface. Subsequently, the tip opening portion of the pipette is moved away from the culture surface, and the drop is separated from the tip opening portion of the pipette.

The contact angle of water on the culture surface of the cell culture container is preferably 40° to 120° at 25°C. Within the above range, the extracellular matrix solution does not spread on the culture surface and is easily placed in a dome shape.

Subsequently, the extracellular matrix solution is gelated to form a cell-containing extracellular matrix. The gelation of the extracellular matrix solution is generally carried out by an operation such as heating or irradiation with light.

At the time when the drop is placed on the culture surface of the cell culture container, the temperature of the culture surface of the cell culture container is preferably 20°C to 60°C and more preferably 20°C to 40°C. Within the above range, the extracellular matrix solution can be rapidly gelated after the extracellular matrix solution is placed and before cells and the like contained in the extracellular matrix solution are sedimented, and as a result, it is possible to obtain a cell-containing extracellular matrix where the cells and the like are uniformly dispersed in the inside of the extracellular matrix.

The shape of the cell-containing extracellular matrix is preferably a dome shape having the culture surface of the culture container as the bottom surface. The dome shape has a hemispherical shape or a shape close thereto. In addition, the volume of the cell-containing extracellular matrix is generally 1 µL to 30 µL and preferably 5 µL to 20 µL. In a case of assuming a hemispherical shape having the same volume as the cell-containing extracellular matrix, the diameter of the hemisphere is generally 2 mm to 6 mm and preferably 2.5 mm to 5 mm.

In a case where the size of the cell-containing extracellular matrix is within the above range, oxygen, carbon dioxide, and nutritional components from the culture medium can be sufficiently supplied to the inside of the extracellular matrix, and thus the proliferation or differentiation of cells can be carried out favorably.

Examples of the cell culture container include a well plate. Examples of the well plate include a 6-well plate, a 12-well plate, a 24-well plate, a 48-well plate, and a 96-well plate. In a case where the cell culture container is a well plate, the bottom surface of the well is the culture surface. Examples of the shape of the bottom surface of the well include a U-shape, a V-shape, and a planar shape. Among these, a planar shape is preferable. Further, the culture surface of the cell incubator can be subjected to cell non-adhesiveness treatment or embossing with a 2-methacryloyloxyethyl phosphorylcholine polymer or the like.

After the production method according to the present embodiment, a culture medium can be brought into contact with the formed cell-containing extracellular matrix. In this case, the present embodiment is a cell culture method. The culture medium can be appropriately selected depending on the intended purpose. In a case where the cell culture container is a well plate, the culture medium can be brought into contact with the cell-containing extracellular matrix by putting the culture medium in the well.

According to the production method according to the present embodiment, it is possible to suspend cells and the like before the extracellular matrix solution is sucked into the inside of a pipette having a tip opening portion. The suspension of cells and the like can be carried out by repeating the operation of sucking the extracellular matrix solution containing cells and the like into the inside of a pipette having a tip opening portion and discharging the cells or the like.

At this time, it is preferable to hold the tip opening portion of the pipette at the bottom part of the container accommodating the extracellular matrix solution. In addition, from the viewpoint of suppressing air from mixedly entering the inside of the pipette, it is preferable that the extracellular matrix solution be sucked into the inside of the pipette while maintaining a state where the tip opening portion of the pipette is below the solution surface of the extracellular matrix solution, and discharged

The pipette for cell suspension to be used at the time of suspending cells and the pipette for forming a drop to be used at the time of forming a drop of the extracellular matrix solution and placing it on the culture surface of the cell culture container may be the same or may be different from each other.

### [Device for Producing Cell-containing Extracellular Matrix]

A device for producing a cell-containing extracellular matrix according to one embodiment includes an accommodation tank that accommodates an extracellular matrix solution in which cells or a cell mass is suspended; a pipette control unit including a nozzle to which a pipette having a tip opening portion is mounted, a pump that applies positive pressure or negative pressure to the inside of the nozzle, a transfer unit that transfers the nozzle, and a control unit that controls operations of the nozzle, the pump, and the transfer unit; and a stage that holds a cell culture container.

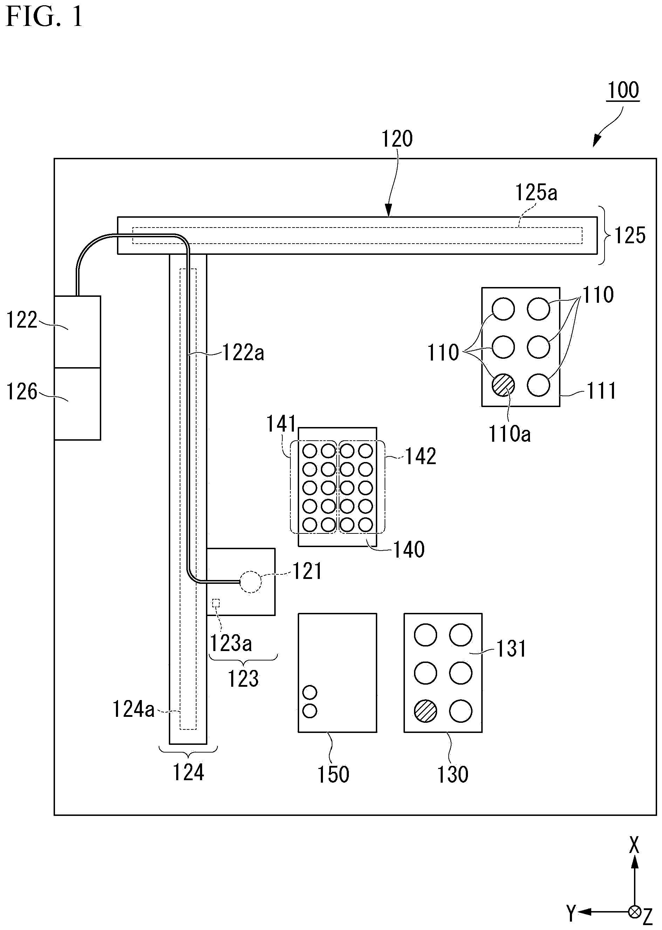

FIG. 1 is a plan view of a device for producing a cell-containing extracellular matrix 100 according to the present embodiment. The device for producing a cell-containing extracellular matrix 100 according to one embodiment includes an accommodation tank 110 that accommodates an extracellular matrix solution in which cells or a cell mass is suspended; a pipette control unit 120 including a nozzle 121 to which a pipette having a tip opening portion is mounted, a pump 122 that applies positive pressure or negative pressure to the inside of the nozzle 121, a transfer unit that transfers the nozzle 121, and a control unit 126 that controls operations of the nozzle 121, the pump 122, and the transfer unit; and a stage 130 that holds a cell culture container 131. The transfer unit includes a vertical transfer unit 123, a front-rear transfer unit 124, and a left-right transfer unit 125.

In the following description, the front-rear direction is referred to as the "X axis", the left-right direction is referred to as the "Y axis", and the height direction of the device for producing a cell-containing extracellular matrix 100 orthogonal to the X axis and the Y axis is referred to as "Z axis" in FIG. 1.

Specific exemplary examples of the accommodation tank 110 include a plastic tube accommodated in an accommodation tank rack 111. In a case where the extracellular matrix solution is an extracellular matrix solution that is gelated in response to temperature, the temperature of the extracellular matrix solution accommodated in the accommodation tank can be maintained at a temperature at which it is not gelated, for, for example, 0°C to 10°C by providing a temperature control function in the accommodation tank rack 111.

In the example of FIG. 1, one of a plurality of accommodation tanks 110 accommodates an extracellular matrix solution 110a in which cells and the like are suspended. As shown in FIG. 1, the pipette control unit 120 includes the nozzle 121, the pump 122, the vertical transfer unit 123, the front-rear transfer unit 124, the left-right transfer unit 125, and the control unit 126. As shown in FIG. 1, the device for producing the cell-containing extracellular matrix 100 can further include a trash unit 150 in which a used pipette is discarded.

The pipette 141 or a pipette 142 having a tip opening portion is attachable and detachable to the nozzle 121. In the example of FIG. 1, a pipette rack 140 accommodates the pipettes 141 and 142. In the example of FIG. 1, the pipette 141 is a pipette for cell suspension, and the pipette 142 is a pipette for forming a drop.

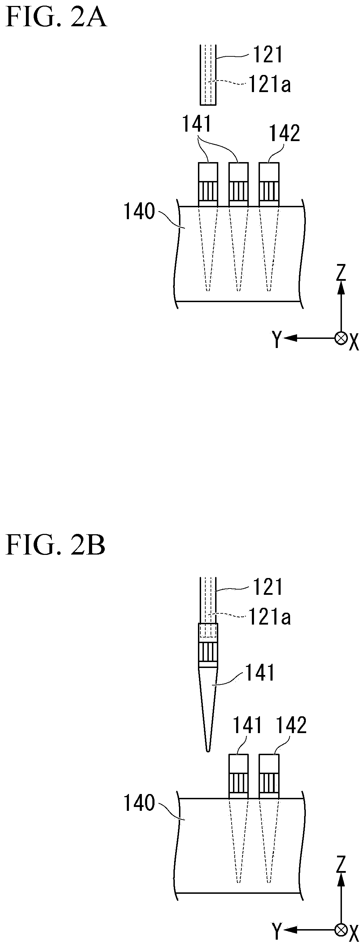

FIGS. 2A and 2B are each a side view of the pipette rack 140. As shown in FIGS. 2A and 2B, in a case where the nozzle 121 of the pipette control unit 120 is lowered from directly above the pipette 141, the pipette 141 is mounted at the lower end of the nozzle 121. Then, in a case where the nozzle 121 rises upward, the pipette 141 is pulled out from the pipette rack 140. The nozzle 121 has a hole 121a so that a liquid can be sucked and discharged from the lower end of the nozzle 121.

The nozzle 121 includes a motor and can be configured to eliminate the pipette 141 or 142 mounted on the nozzle 121 based on a signal from the control unit 126.

The pump 122 is connected to the hole 121a of the nozzle 121 via a tube 122a. The pump 122 applies positive pressure and negative pressure to the hole 121a of the nozzle 121 based on the signal from the control unit 126 and sucks or discharges the extracellular matrix solution 110a via the pipette 141 or 142 mounted on the lower end of the nozzle 121.

The vertical transfer unit 123 includes a rail 123a extending along the Z axis, and a motor. The vertical transfer unit 123 drives the motor based on the signal from the control unit 126 and transfers the nozzle 121 along the rail 123a in the Z-axis direction.

The front-rear transfer unit 124 includes a rail 124a extending along the X axis, and a motor. The front-rear transfer unit 124 drives the motor based on the signal from the control unit 126 and transfers the vertical transfer unit 123 along the rail 124a in the X axis direction.

The left-right transfer unit 125 includes a rail 125a extending along the Y-axis, and a motor. The left-right transfer unit 125 drives the motor based on the signal from the control unit 126 and transfers the front-rear transfer unit 124 along the rail 125a in the Y axis direction.

In this way, the nozzle 121 can be moved along the XYZ axes in the inside of the device for producing the cell-containing extracellular matrix 100 by the vertical transfer unit 123, the front-rear transfer unit 124, and the left-right transfer unit 125.

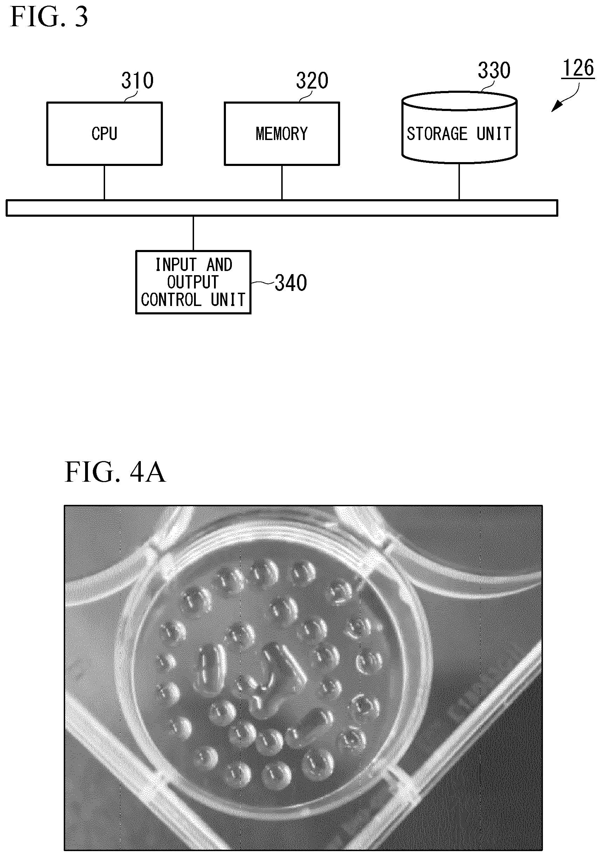

FIG. 3 is a block diagram showing a configuration of the control unit 126. As shown in FIG. 3, the control unit 126 is a device (a computer) that can execute a program, including a central processing unit (CPU) 310, a memory 320, a storage unit 330, and an input and output control unit 340. It functions as a plurality of functional blocks by executing a predetermined program. It should be noted that at least some of the functions of the control unit 126 can be constituted by a single-purpose logic circuit or the like.

The storage unit 330 is a non-volatile recording medium that stores the above-described program and necessary data. The storage unit 330 is composed of, for example, a ROM, a hard disk, or the like. The program recorded in the storage unit 330 is read into the memory 320 and executed by the CPU 310.

The input and output control unit 340 generates control signals for the nozzle 121, the pump 122, the vertical transfer unit 123, the front-rear transfer unit 124, the left-right transfer unit 125, and the like based on the instruction of the CPU 310.

A temperature control function for a heater, a cooler, or the like can be provided in the accommodation tank rack 111 in which the accommodation tank 110 is accommodated. The accommodation tank rack 111 may further include a temperature sensor. Further, a temperature control function for a heater, a cooler, or the like can be provided in the stage 130 holding the cell culture container 131. The stage 130 may further include a temperature sensor.

In addition, the input and output control unit 340 can be configured to receive data from each of the above-described temperature sensors. Further, the input and output control unit 340 can be configured to generate control signals for a heater and a cooler based on the instruction of the CPU 310. This makes it possible to control the temperature of the cell-containing extracellular matrix solution accommodated in the accommodation tank 110 and the temperature of the culture surface of the cell culture container 131 held in the stage 130 to a predetermined temperature.

### [Control Program for Cell-containing Extracellular Matrix-Producing Device]

A control program for a cell-containing extracellular matrix-producing device according to the present embodiment causing for a cell-containing extracellular matrix-producing device to form a cell-containing extracellular matrix on a culture surface of a cell culture container, the cell-containing extracellular matrix-producing device including an accommodation tank that accommodates an extracellular matrix solution in which cells or a cell mass is suspended, a pipette control unit including a nozzle to which a pipette having a tip opening portion is mounted, a pump that applies positive pressure or negative pressure to the nozzle, a transfer unit that transfers the nozzle, and a control unit that controls operations of the nozzle, the pump, and the transfer unit, and a stage that holds a cell culture container, in which in the device for producing a cell-containing extracellular matrix in which an extracellular matrix solution in which cells or a cell mass is suspended is accommodated in the accommodation tank, a pipette having a tip opening portion is mounted on the nozzle, and the cell culture container is placed on the stage, in which the control unit is caused to control the transfer unit to move a tip of the pipette mounted on the nozzle below a solution surface of the extracellular matrix solution accommodated in the accommodation tank, the control unit is caused to control the pump to suck the extracellular matrix solution into the inside of the pipette, the control unit is caused to control the transfer unit to move the pipette that has sucked the extracellular matrix solution to an upper part of the cell culture container, the control unit is caused to control the pump to discharge the extracellular matrix solution in the inside of the pipette to form a drop of the extracellular matrix solution at the tip opening portion of the pipette, the control unit is caused to control the transfer unit to bring the tip opening portion of the pipette close to the culture surface of the cell culture container and to place the drop on the culture surface while avoiding bringing the tip opening portion of the pipette into contact with the culture surface, the control unit is caused to control the transfer unit to move the tip opening portion of the pipette away from the culture surface, to separate the drop from the tip opening portion of the pipette, and the extracellular matrix solution is subsequently gelated to form a cell-containing extracellular matrix.

By the program of the present embodiment, it is possible for the above-described device for producing a cell-containing extracellular matrix 100 to produce a cell-containing extracellular matrix. Hereinafter, a description will be made for each step of producing a cell-containing extracellular matrix by controlling the device for producing a cell-containing extracellular matrix 100 by the program according to the present embodiment.

An extracellular matrix solution in which cells or a cell mass is suspended is accommodated in the accommodation tank 110 of the above-described device for producing the cell-containing extracellular matrix 100. In addition, the cell culture container 131 is placed on the stage 130. These operations may be carried out by the operator's manual operation.

Subsequently, the pipette 141 is mounted on the nozzle 121. The mounting of the pipette 141 on the nozzle 121 can be carried out by the operator's manual operation or may be carried out by the control unit 126 of the device for producing a cell-containing extracellular matrix. In this case, first, the control unit 126 controls the vertical transfer unit 123, the front-rear transfer unit 124, and the left-right transfer unit 125 to move the nozzle 121 of the pipette control unit 120 to the pipette rack 140. Subsequently, the nozzle 121 is lowered from directly above the pipette 141. As a result, the pipette 141 is mounted at the lower end of the nozzle 121. Subsequently, the nozzle 121 is raised upward, and the pipette 141 is pulled out from the pipette rack 140.

As necessary, the extracellular matrix solution accommodated in the accommodation tank 110 can be suspended. The control unit 126 controls the vertical transfer unit 123, the front-rear transfer unit 124, and the left-right transfer unit 125 to move the tip of the pipette 141 mounted on the nozzle 121 to the upper part of the accommodation tank 110.

The tip of the pipette 141 mounted on the nozzle 121 is lowered to be moved below the solution surface of the extracellular matrix solution accommodated in the accommodation tank 110. The control unit 126 controls the pump 122 to suck the extracellular matrix solution 110a into the inside of the pipette 141 and discharge it, and this operation is repeated a plurality of times. By this operation, the cell mass or cells in the extracellular matrix solution accommodated in the accommodation tank 110 are suspended.

Subsequently, the control unit 126 controls the vertical transfer unit 123, the front-rear transfer unit 124, and the left-right transfer unit 125 to move the pipette 141 mounted on the nozzle 121 to the upper part of the trash unit 150.

The control unit 126 controls the nozzle 121 to eliminate the pipette 141 mounted on the nozzle 121, whereby the used pipette 141 is accommodated in the trash unit 150.

Subsequently, the extracellular matrix solution 110a is sucked into the inside of the pipette 142. First, the pipette 142 is mounted on the nozzle 121. Here, the mount of the pipette 142 on the nozzle 121 can be carried out by the operator's manual operation or may be carried out by the control unit 126 of the device for producing a cell-containing extracellular matrix. In this case, first, the control unit 126 controls the vertical transfer unit 123, the front-rear transfer unit 124, and the left-right transfer unit 125 to move the nozzle 121 of the pipette control unit 120 to the pipette rack 140. Subsequently, the nozzle 121 is lowered from directly above the pipette 142. As a result, the pipette 142 is mounted at the lower end of the nozzle 121. Subsequently, the nozzle 121 is raised upward, and the pipette 142 is pulled out from the pipette rack 140.

The control unit 126 controls the vertical transfer unit 123, the front-rear transfer unit 124, and the left-right transfer unit 125 to move the nozzle 121 of the pipette control unit 120 to the upper part of the accommodation tank 110.

The tip of the pipette 142 mounted on the nozzle 121 is lowered to be moved below the solution surface of the extracellular matrix solution accommodated in the accommodation tank 110. Subsequently, the control unit 126 controls the pump 122 to suck the extracellular matrix solution 110a into the inside of the pipette 142.

At this time, a lid is removed in a case where the cell culture container 131 is covered with the lid. The lid may be removed by the operator's manual operation or may be removed by the control unit 126 of the device for producing a cell-containing extracellular matrix. In a case where the lid is removed by the control unit 126, for example, an arm for gripping the lid can be configured to be arranged next to the nozzle 121, and then the vertical transfer unit 123, the front-rear transfer unit 124, and the left-right transfer unit 125 can be configured to control the position of the arm, thereby causing the control unit 126 to control the gripping and releasing of the lid by the arm.

Subsequently, the control unit 126 controls the vertical transfer unit 123, the front-rear transfer unit 124, and the left-right transfer unit 125 to move the pipette 142 that has been mounted on the nozzle 121 and has sucked the extracellular matrix solution 110a to the upper part of the cell culture container 131.

Subsequently, the control unit 126 controls the pump 122 to discharge the extracellular matrix solution 110a inside the pipette 142, whereby a drop of the extracellular matrix solution 110a is formed at the tip opening portion of the pipette 142.

Subsequently, the control unit 126 controls the vertical transfer unit 123, the front-rear transfer unit 124, and the left-right transfer unit 125 to cause the tip opening portion of the pipette 142 to be brought close to the culture surface of the cell culture container 131, and the drop is placed on the culture surface while avoiding bringing the tip opening portion of the pipette 142 into contact with the culture surface.

Subsequently, the control unit 126 controls the vertical transfer unit 123, the front-rear transfer unit 124, and the left-right transfer unit 125 to move the tip opening portion of the pipette 142 away from the culture surface, whereby the drop is separated from the tip opening portion of the pipette 142. The extracellular matrix solution 110a is gelated by heating, irradiation with light, or the like, depending on the characteristics of the extracellular matrix solution 110a used, to form a cell-containing extracellular matrix.

By repeating as many times as necessary the operation of moving the pipette 142, forming a drop, placing the drop on the culture surface, and separating the drop from the tip opening portion of the pipette 142 described above, it is possible to form a plurality of cell-containing extracellular matrices having a uniform size and shape on the culture surface of the cell culture container 131.

Subsequently, the control unit 126 controls the vertical transfer unit 123, the front-rear transfer unit 124, and the left-right transfer unit 125 to move the pipette 142 mounted on the nozzle 121 to the upper part of the trash unit 150.

The control unit 126 controls the nozzle 121 to eliminate the pipette 142 mounted on the nozzle 121, whereby the used pipette 142 is accommodated in the trash unit 150.

Subsequently, as necessary, the operation of mounting the pipette 141 on the nozzle 121, suspending the extracellular matrix solution accommodated in the accommodation tank 110, accommodating the pipette 141 in the trash unit 150, mounting the pipette 142 on the nozzle 121, sucking the extracellular matrix solution 110a in the inside of the pipette 142, moving the pipette 142, forming a drop, placing the drop on the culture surface, and separating the drop from the tip opening portion of the pipette 142 as described above is repeated to produce the required number of cell-containing extracellular matrices.

The production may be realized by recording the program in the above-described embodiment on a computer-readable recording medium and causing a computer system to read the program recorded on the recording medium, thereby executing the program. It should be noted that the term "computer system" referred to here includes hardware such as an OS and peripheral devices. Further, the term "computer-readable recording medium" refers to a portable medium such as a flexible disk, a magneto-optical disk, a ROM, or a CD-ROM, and a storage device such as a hard disk that is incorporated into a computer system. Further, the term "computer-readable recording medium" may also include those in which a program is dynamically held for a short period of time, such as a communication line in a case of transmitting a program via a network such as the Internet or a communication line such as a telephone line, and in which a program is held for a certain time, such as a volatile memory in the inside of a computer system that is a server or a client in that case. Further, the above program may be a program for realizing some of the above-described functions, and further, it may be a program that can realize the above-described functions in combination with a program already recorded in the computer system or a program that is realized by using a programmable logic device such as a field programmable gate array (FPGA).

### Examples

Hereinafter, the present embodiment will be described in more detail based on Examples. However, the present embodiment is not limited to these Examples.

### [Experimental Example 1]

A cell-containing extracellular matrix was produced using the device for producing a cell-containing extracellular matrix of the embodiment. In addition, a cell-containing extracellular matrix was produced and compared by the operator's manipulation for comparison.

As the device for producing a cell-containing extracellular matrix, a device programmed with a laboratory automation system (product name "Biomek i5", Beckman Coulter, Inc.) was used.

As the extracellular matrix solution, Matrigel (Corning Incorporated) in which human iPS cell-derived intestinal epithelial cells were suspended at a cell density of 2 × 10⁵ cells/mL was used. The temperature of the extracellular matrix solution accommodated in the accommodation tank was maintained at 6°C.

As the cell culture container, a 6-well plate (Greiner Bio-One Inc.) was used. The contact angle of water on the culture surface of this 6-well plate was 53° at 25°C. The temperature of the culture surface of the cell culture container was maintained at 37°C.

As the pipette for forming a drop, an AP96 P250 tip (Beckman Coulter, Inc.) was used. The opening area of the tip opening portion of the pipette for forming a drop was 0.28 mm².

FIG. 4A and FIG. 6A are each a photographic image showing the result of placing drops of a cell-containing extracellular matrix solution in a well of a 6-well plate by an operator's manipulation. FIG. 4B and FIG. 6B are each a photographic image showing the result of placing drops of the cell-containing extracellular matrix solution in a well of a 6-well plate using the device for producing a cell-containing extracellular matrix. The operator's manipulation indicates a method in which a skilled person forms a cell-containing extracellular matrix at the same speed as the device for producing an extracellular matrix by using the same cell culture container and the same pipette for forming a drop as the device for producing an extracellular matrix.

The cell-containing extracellular matrix solution placed on the culture surface was then gelated, thereby obtaining a cell-containing extracellular matrix having the same volume as the drop of the cell-containing extracellular matrix solution.

As shown in FIG. 4A or FIG. 6A, as a result of placing drops of a cell-containing extracellular matrix solution in a well of a 6-well plate by an operator's manipulation, drops of the extracellular matrix solution could be placed at about 31 seconds/well. However, although the number of drops per well was about 30, a plurality of gels was linked. In addition, a clear variation in size was observed.

In the drop formed at the tip opening portion of the pipette by the operator's manipulation, the ratio of the maximum value of the length in the horizontal direction to the maximum value of the length in the vertical direction (the maximum value of the length in the horizontal direction/the maximum value of the length in the vertical direction) was 2.45. Further, the maximum value of the length of the drop in the vertical direction was 1.29 mm. Further, in a case of assuming a hemispherical shape having the same volume as the drop of the cell-containing extracellular matrix solution per drop, the diameter of the hemisphere was 2.85 mm.

On the other hand, as shown in FIG. 4B or FIG. 6B, as a result of placing drops of a cell-containing extracellular matrix solution in a well of a 6-well plate by using the device for producing a cell-containing extracellular matrix, drops of the extracellular matrix solution could be placed at about 25 seconds/well. The number of drops per well was 27, and the size thereof was uniform.

In the drop formed at the tip opening portion of the pipette by the device for producing a cell-containing extracellular matrix, the ratio of the maximum value of the length in the horizontal direction to the maximum value of the length in the vertical direction (the maximum value of the length in the horizontal direction/the maximum value of the length in the vertical direction) was 2. Further, the maximum value of the length of the drop in the vertical direction was 1.44 mm. Further, in a case of assuming a hemispherical shape having the same volume as the drop of the cell-containing extracellular matrix solution per drop, the diameter of the hemisphere was 3.05 mm.

FIG. 6C is a photographic image showing the result of discharging the cell-containing extracellular matrix solution in the vicinity of a well of a 6-well plate to place a drop on the well without forming a drop of the cell-containing extracellular matrix solution at the tip opening portion of a pipette, although the device for producing a cell-containing extracellular matrix was used. The extracellular matrix solution could be placed at about 25 seconds/well, and the number of drops per well was 27; however, there were 4 drops bound to adjacent drops.

Table 1 below shows, the number of drops placed on the well (indicated as "Number of drops on well" in Table 1), the number of drops bound to the adjacent drops (indicated as "Number of bound drops" in Table 1), the average value of the diameter of the bottom surface of the drops in a case of regarding the drops as hemispherical (indicated as "Average diameter of drops" in Table 1) and the standard deviations thereof in FIG. 6A to FIG. 6C.

### [Experimental Example 2]

The culture medium was put in the wells as shown in the photographic images of FIG. 6A to FIG. 6C, and the culture was carried out at 37°C for 7 days to obtain cultured cells. As the culture medium, a culture medium obtained by adding a niche factor such as Wnt3a to a basal culture medium (product name "Advanced DMEM/F12", manufactured by Thermo Fisher Scientific, Inc.) was used. Then, the average size of the obtained cultured cells was measured. Table 1 below shows the average size of the measured cultured cells.

**[Table 1]**

| Item | FIG. 6A | FIG. 6B | FIG. 6C |
|---|---|---|---|
| Number of drops on well (drops) | 33 | | |
| | | | |
| | | | |
| | | | 0.2 |
| Average size of cultured cells (µm³) | 257,988 | 644,844 | 244,844 |

### Industrial Applicability

According to the present embodiment, it is possible to produce a plurality of extracellular matrices having a uniform size and shape by merely changing the program of the existing pipetting device. As a result, according to the present embodiment, it is possible to provide a method for producing a cell-containing extracellular matrix which is suitable for mechanical automation.

### [Reference Signs List]

100: Cell-containing extracellular matrix-producing device
110: Accommodation tank
110a: Extracellular matrix solution
111: Accommodation tank rack
120: Pipette control unit
121: Nozzle
121a: Hole
122: Pump
122a: Tube
123: Vertical transfer unit
124: Front-rear transfer unit
125: Left-right transfer unit
123a, 124a, 125a: Rail
126: Control unit
130: Stage
131: Cell culture container
140: Pipette rack
141,142: Pipette
141a: Tip opening portion of pipette
150: Trash unit
320: Memory
330: Storage unit
340: Input and output control unit
500: Drop.

## Claims

1. A method for producing a cell-containing extracellular matrix, the method comprising:
preparing an extracellular matrix solution which contains (i) an extracellular matrix precursor and (ii) cells or a cell mass inside a pipette having a tip opening portion;
discharging the extracellular matrix solution to form a drop of the extracellular matrix solution at the tip opening portion of the pipette;
bringing the tip opening portion of the pipette close to a culture surface of a cell culture container to place the drop on the culture surface while avoiding bringing the tip opening portion of the pipette into contact with the culture surface;
moving the tip opening portion of the pipette away from the culture surface to separate the drop from the tip opening portion of the pipette; and
gelating the extracellular matrix solution to form a cell-containing extracellular matrix.

2. The method for producing a cell-containing extracellular matrix according to claim 1,
wherein a contact angle of water on the culture surface is 40° to 120° at 25°C.

3. The method for producing a cell-containing extracellular matrix according to claim 1 or 2,
wherein at a time of forming the cell-containing extracellular matrix, a temperature of the culture surface is 20°C to 60°C.

4. The method for producing a cell-containing extracellular matrix according to any one of claims 1 to 3,
wherein a temperature of the extracellular matrix solution is 0°C to 10°C.

5. The method for producing a cell-containing extracellular matrix according to any one of claims 1 to 4,
wherein an opening area of the tip opening portion of the pipette is 0.05 mm² to 0.4 mm².

6. The method for producing a cell-containing extracellular matrix according to any one of claims 1 to 5,
wherein in the drop, a ratio of a maximum value of a length in a horizontal direction to a maximum value of a length in a vertical direction (the maximum value of the length in the horizontal direction/the maximum value of the length in the vertical direction) is 0.5 to 5.

7. The method for producing a cell-containing extracellular matrix according to any one of claims 1 to 6,
wherein a maximum value of a length of the drop in a vertical direction is 100 µm to 5,000 µm.

8. The method for producing a cell-containing extracellular matrix according to any one of claims 1 to 7,
wherein the cell culture container is a well plate.

9. The method for producing a cell-containing extracellular matrix according to any one of claims 1 to 8,
wherein at a time of preparing the extracellular matrix solution, sucking the extracellular matrix solution into the inside of the pipette while maintaining a state where the tip opening portion of the pipette is below a solution surface of the extracellular matrix solution.

10. A cell culture method comprising bringing a cell-containing extracellular matrix formed according to the method for producing a cell-containing extracellular matrix according to any one of claims 1 to 9 into contact with a culture medium and culturing the cells.

11. A device for producing a cell-containing extracellular matrix, the device comprising:
an accommodation tank that accommodates an extracellular matrix solution containing (i) an extracellular matrix precursor and (ii) cells or a cell mass;
a pipette control unit including a nozzle to which a pipette having a tip opening portion is mounted, a pump that applies positive pressure or negative pressure to a hole of the nozzle, a transfer unit that transfers the nozzle, and a control unit that controls operations of the nozzle, the pump, and the transfer unit; and
a stage that holds a cell culture container.

12. A control program for a cell-containing extracellular matrix-producing device, causing a cell-containing extracellular matrix-producing device to form a cell-containing extracellular matrix on a culture surface of a cell culture container, the cell-containing extracellular matrix-producing device including;
an accommodation tank that accommodates an extracellular matrix solution containing (i) an extracellular matrix precursor and (ii) cells or a cell mass;
a pipette control unit including a nozzle to which a pipette having a tip opening portion is mounted, a pump that applies positive pressure or negative pressure to the nozzle, a transfer unit that transfers the nozzle, and a control unit that controls operations of the nozzle, the pump, and the transfer unit, and
a stage that holds a cell culture container,
wherein in the device for producing a cell-containing extracellular matrix in which the extracellular matrix solution containing (i) an extracellular matrix precursor and (ii) cells or a cell mass is accommodated in the accommodation tank, a pipette having a tip opening portion is mounted on the nozzle, and the cell culture container is placed on the stage,
wherein the control unit is caused to control the transfer unit to move a tip of the pipette mounted on the nozzle below a solution surface of the extracellular matrix solution accommodated in the accommodation tank,
the control unit is caused to control the pump to suck the extracellular matrix solution into the inside of the pipette,
the control unit is caused to control the transfer unit to move the pipette that has sucked the extracellular matrix solution to an upper part of the cell culture container,
the control unit is caused to control the pump to discharge the extracellular matrix solution in the inside of the pipette to form a drop of the extracellular matrix solution at the tip opening portion of the pipette,
the control unit is caused to control the transfer unit to bring the tip opening portion of the pipette close to the culture surface of the cell culture container and to place the drop on the culture surface while avoiding bringing the tip opening portion of the pipette into contact with the culture surface,
the control unit is caused to control the transfer unit to move the tip opening portion of the pipette away from the culture surface, to separate the drop from the tip opening portion of the pipette, and
the extracellular matrix solution is subsequently gelated to form a cell-containing extracellular matrix.
